# EUROPÄISCHE PATENTSCHRIFT

(11) **EP 3 057 568 B1**
(45) Veröffentlichungstag und Bekanntmachung des Hinweises auf die Patenterteilung: **18.07.2018**
(21) Anmeldenummer: 14796689.9
(22) Anmeldetag: 26.09.2014
(51) Int. Cl.: A61K 8/26, A61K 8/46, A61Q 15/00

(54) **VERWENDUNG VON METHANSULFONSÄURE UND IHRER SALZE ZUR AKTIVIERUNG UND/ODER STABILISIERUNG SCHWEISSHEMMENDER ALUMINIUMSALZE**
USE OF METHANESULFONIC ACID AND ITS SALTS FOR ACTIVATING AND/OR STABILIZING ANTIPERSPIRANT ALUMINIUM SALTS
UTILISATION DE L'ACIDE MÉTHANESULFONIQUE ET DE SES SELS POUR L'ACTIVATION ET/OU STABILISATION DES SELS D'ALUMINIUM ANTI-TRANSPIRANTS

(30) Priorität: 15.10.2013 DE 102013220783
(43) Veröffentlichungstag der Anmeldung: 24.08.2016
(73) Patentinhaber: Henkel AG & Co. KGaA, 40589 Düsseldorf (DE)
(72) Erfinder: BANOWSKI, Bernhard, 40597 Düsseldorf (DE); CLAAS, Marcus, 40723 Hilden (DE)
(86) Internationale Anmeldenummer: PCT/DE2014/200517
(87) Internationale Veröffentlichungsnummer: WO 2015/055200

(56) Entgegenhaltungen:
- WO-A2-2007/059530
- WO-A2-2014/095685
- WO-A2-2014/095688
- DE-A1- 2 249 580
- US-A- 3 953 450

## Beschreibung

Die vorliegende Erfindung betrifft die Verwendung einer Alkylsulfonsäure zur Aktivierung und/oder Stabilisierung eines schweißhemmenden Aluminiumsalzes.

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher).

Kosmetische Antitranspirantien des Standes der Technik enthalten neben mindestens einem Öl oder einer Fettsubstanz und einer Riechstoffkomponente bzw. einem Parfüm mindestens ein schweißhemmendes Salz.

Als schweißhemmendes Salz werden für gewöhnlich basische Aluminium- und Aluminium-Zirkoniumhalogenide in Form von Chloriden eingesetzt, da sie im Unterschied zu den nichtbasischen Aluminium- und Aluminium-Zirkoniumchloriden keine hautreizende Wirkung aufweisen. Der Nachteil der basischen Aluminium- und Aluminium-Zirkoniumhalogenide liegt jedoch in der Bildung von höhermolekularen oligomeren und polymeren Aluminium-Spezies, welche die Wirksamkeit der basischen Aluminium- und Aluminium-Zirkoniumsalze in Antitranspirantien signifikant reduzieren.

Aus diesem Grund wurde im Stand der Technik versucht, die Wirksamkeit der basischen Aluminium- und Aluminium-Zirkoniumhalogenide durch Aktivierung zu steigern, jedoch gleichzeitig die gute Hautverträglichkeit beizubehalten. So sind in den Druckschriften EP 0 308 937 A2, EP 0 183 171 A2, US 4 359 456 A und EP 0 191 628 A2 basische Aluminium- und Aluminium-Zirkoniumhalogenide, insbesondere Aluminiumchloride, beschrieben, welche durch eine thermischen Behandlung erhalten wurden. Die wärmebehandelten aktivierten basischen Aluminium- und Aluminium-Zirkoniumhalogenide weisen in der Gelpermeationschromatographie (GPC) einen geringeren Anteil an hochmolekularen Spezies im Vergleich zu unbehandelten basischen Aluminium- und Aluminium-Zirkoniumhalogeniden und somit eine gesteigerte Wirksamkeit gegen Schweißsekretion in Antitranspirantien auf.

Weiterhin kann die Wirksamkeit basischer Aluminium- und Aluminium-Zirkoniumhalogenide gesteigert werden, indem organische Säuren als Komplexliganden eingebaut werden. So werden in den Druckschriften US 3 542 919 A, US 3 553 316 A, US 3 991 176 A und WO 2005/092795 A1 Verfahren zur Herstellung derartiger stabilisierter Aluminium- und Aluminium-Zirkoniumhalogenide offenbart, welche einen höheren Anteil an kurzkettigen Spezies aufweisen und in aktivierter Form vorliegen. Weiterhin betrifft die Druckschrift WO 2007/059530 A2 die Verwendung einer Kombination von Hydroxysäuren und zwitterionischen Verbindungen zur Aktivierung von Aluminium- und/oder Aluminium-Zirkoniumsalzen.

Ein Nachteil der vorgenannten aktivierten basischen Aluminium- und Aluminium-Zirkoniumhalogenide des Standes der Technik besteht in der Abnahme der schweißhemmenden Wirkung mit zunehmender Lagerungsdauer und/oder bei Einsatz von protischen Lösungsmitteln. Dies liegt darin begründet, dass die Polymerverteilung der Aluminium-Spezies auf einem reversiblen Gleichgewicht basiert und sich dieses Gleichgewicht in protischen Lösungsmitteln mit zunehmender Zeitdauer zu höhermolekularen und damit weniger wirksamen Aluminium-Spezies verschiebt.

Es besteht daher ein Bedarf an Verbindungen, welche selbst während langen Lagerungszeiträumen und/oder bei der Verwendung von hohen Mengen an protischen Lösungsmitteln eine Verschiebung der Polymerverteilung zu langkettigen Polymeren und somit eine signifikante Abnahme der schweißhemmenden Wirkung von Aluminium- und/oder Aluminium-Zirkoniumsalzen vermindern bzw. vermeiden.

Der vorliegenden Erfindung lag die Aufgabe zugrunde, Verbindungen bereitzustellen, welche eine signifikante Abnahme der Aktivierung von schweißhemmenden Aluminiumsalzen während längeren Lagerungszeiträumen und/oder in Anwesenheit von protischen Lösungsmitteln vermindern bzw. vermeiden.

Es wurde nun überraschend gefunden, dass der Einsatz von Alkylsulfonsäuren bzw. deren Salzen zu einer signifikant erhöhten Bildung von kurzkettigen Aluminium-Species sowie zu einer effektiven Stabilisierung dieser kurzkettigen Aluminium-Species selbst über lange Lagerungszeiträume führt.

Gegenstand der vorliegenden Erfindung ist somit die Verwendung mindestens einer Verbindung der Formel (S-I) 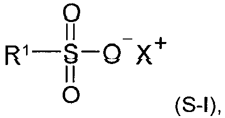 worin X⁺ für H⁺, Li⁺, Na⁺, K⁺, NH₄⁺, oder ½ Mg²⁺ steht, R¹ für eine Methyl-Gruppe steht,
zur Aktivierung und/oder Stabilisierung mindestens eines schweißhemmenden Aluminiumsalzes. Unter Aktivierung ist im Sinne der vorliegenden Erfindung die signifikant vermehrte Bildung von kurzkettigen Polymeren des schweißhemmenden Aluminiumsalzes bei Verwendung der Verbindung der Formel (S-I) zu verstehen.

Weiterhin ist unter Stabilisierung im Sinne der vorliegenden Erfindung die Vermeidung bzw. die signifikante Verlangsamung der erneuten Bildung von höherkettigen Polymeren aus den bei der Aktivierung gebildeten kurzkettigen Polymeren der schweißhemmenden Aluminiumsalze zu verstehen.

Unter dem Begriff "schweißhemmend" wird erfindungsgemäß die Verminderung bzw. Reduzierung der Transpiration der Schweißdrüsen des Körpers verstanden.

Durch den Einsatz von speziellen Sulfonsäuren der Formel (S-I) ist es überraschenderweise gelungen, die Aktivität von schweißhemmenden Aluminiumsalzen selbst über einen längeren Lagerungszeitraum signifikant zu steigern. Ohne sich auf diese Theorie beschränken zu wollen wird durch den Einsatz von Verbindungen der Formel (S-I) eine signifikante Erhöhung des Anteils an kurzkettigen Spezies der schweißhemmenden Aluminiumsalze erreicht. Weiterhin werden die auf diese Weise gebildeten kurzkettigen Spezies durch die vorgenannten Sulfonsäuren hervorragend stabilisiert, so dass eine verbesserte schweißhemmende Wirkung über einen langen Zeitraum gewährleistet wird.

Eine besonders gute Aktivierung und/oder Stabilisierung des mindestens einen schweißhemmenden Aluminiumsalzes wird erreicht, wenn die Verbindung der Formel (S-I) in einer Gesamtmenge von 0,01 bis 50 Gew.-%, vorzugsweise von 0,1 bis 40 Gew.-%, bevorzugt von 0,5 bis 30 Gew.-%, weiter bevorzugt von 1 bis 20 Gew.-%, noch weiter bevorzugt von 1,5 bis 10 Gew.-%, insbesondere von 2 bis 7 Gew.-%, bezogen auf das Gesamtgewicht aus der Verbindung der Formel (S-I), dem mindestens einen schweißhemmenden Aluminiumsalz sowie gegebenenfalls weiteren Inhaltsstoffen, verwendet wird. Die vorstehend genannten Mengen führen zu einer besonders guten Aktivierung und Stabilisierung des schweißhemmenden Aluminiumsalzes selbst über längere Lagerungszeiträume.

In diesem Zusammenhang ist es von Vorteil, wenn das schweißhemmende Aluminiumsalz in einer Gesamtmenge von 1 bis 99,99 Gew.-%, vorzugsweise von 2 bis 80 Gew.-%, bevorzugt von 3 bis 60 Gew.-%, weiter bevorzugt von 4 bis 50 Gew.-%, insbesondere von 6 bis 45 Gew.-%, bezogen auf das Gesamtgewicht aus der Verbindung der Formel (S-I), dem mindestens einen schweißhemmenden Aluminiumsalz sowie gegebenenfalls weiteren Inhaltsstoffen, verwendet wird.

Als besonders vorteilhaft für die Aktivierung und/oder Stabilisierung des mindestens einen schweißhemmenden Aluminiumsalzes hat es sich erwiesen, wenn ein Gewichtsverhältnis des schweißhemmenden Aluminiumsalzes zu der Verbindung der Formel (S-I) von 5.000 : 1 bis 1 : 0,16, vorzugsweise von 1.000 :1 bis 1 : 1, bevorzugt von 100 : 1 bis 1 : 5, weiter bevorzugt von 50: 1 bis 1 :10, noch weiter bevorzugt von 10 : 1 bis 1 : 30, insbesondere von 4 : 1 bis 1 : 50, verwendet wird. Das vorstehend genannte Gewichtsverhältnis bezieht sich dabei auf die Gesamtmenge aller schweißhemmenden Aluminiumsalze sowie aller Verbindungen der Formel (S-I) in dem schweißhemmenden kosmetischen Mittel.

Überraschenderweise resultiert bei Verwendung der Verbindung der Formel (S-I) selbst in Anwesenheit hoher Mengen an protischen Lösungsmitteln, wie beispielsweise Wasser eine signifikant gesteigerte Aktivierung und/oder die Stabilisierung des schweißhemmenden Aluminiumsalzes. Daher ist es bevorzugt, wenn eine Gesamtmenge an freiem Wasser von 1 bis 90 Gew.-%, vorzugsweise von 5 bis 80 Gew.-%, bevorzugt von 10 bis 70 Gew.-%, weiter bevorzugt von 12 bis 60 Gew.-%, insbesondere von 15 bis 55 Gew.-%, bezogen auf das Gesamtgewicht aus der Verbindung der Formel (S-I), dem mindestens einen schweißhemmenden Aluminiumsalz, dem freien Wasser sowie gegebenenfalls weiteren Inhaltsstoffen, verwendet wird.

Weiterhin ist es im Rahmen der vorliegenden Erfindung ebenfalls möglich, die aus der Aktivierung und Stabilisierung des mindestens einen schweißhemmenden Aluminiumsalzes durch die Verbindung der Formel (S-I) resultierende flüssige Mischung zu trocknen. Die Trocknung dieser Mischung kann beispielsweise mittels herkömmlicher Trocknungsverfahren, wie Sprühtrocknung, durchgeführt werden. Die auf diese Weise erhaltenen Pulver lassen sich hervorragend lagern und weisen eine lange Lagerstabilität auf.

Erfindungsgemäß bevorzugt erfolgt die Verwendung der mindestens einen Verbindung der Formel (S-I) in schweißhemmenden kosmetischen Mitteln, enthaltend
a) mindestens einen Stoff, ausgewählt aus der Gruppe von kosmetischen Ölen, welche bei 20 °C und 1.013 hPa flüssig sind, Riechstoffen und Wachsen,
b) mindestens ein schweißhemmendes Aluminiumsalz in einer Gesamtmenge von 1 bis 80 Gew.-%, bezogen auf das Gesamtgewicht des schweißhemmenden kosmetischen Mittels.

Durch die Kombination von schweißhemmenden Aluminiumsalzen mit speziellen Sulfonsäuren der Formel (S-I) wird - ohne sich auf diese Theorie beschränken zu wollen - eine signifikante Erhöhung des Anteils an kurzkettigen Spezies der schweißhemmenden Aluminiumsalze erreicht. Weiterhin werden die auf diese Weise gebildeten kurzkettigen Spezies durch die vorgenannten Sulfonsäuren hervorragend stabilisiert, so dass eine verbesserte schweißhemmende Wirkung über einen langen Zeitraum gewährleistet wird. Durch den Einsatz der speziellen Sulfonsäuren können insbesondere Antitranspirantien mit einem hohen Anteil an protischen Lösungsmitteln hergestellt werden, welche trotz der hohen Mengen an protischen Lösungsmitteln eine hervorragende schweißhemmende Wirkung selbst während längerer Lagerungszeiträume aufweisen.

Unter dem Begriff "kosmetisches Öl" wird im Sinne der vorliegenden Erfindung ein für die kosmetische Verwendung geeignetes Öl verstanden, welches mit Wasser nicht mischbar ist. Weiterhin handelt es sich bei dem erfindungsgemäß verwendeten kosmetischen Öl weder um Riechstoffe, noch um ätherische Öle.

Zudem werden unter dem Begriff "Riechstoffe" im Sinne der vorliegenden Erfindung Substanzen mit einer Molmasse von 74 bis 300 g/mol verstanden, welche mindestens eine osmophore Gruppe im Molekül enthalten und einen Geruch und/oder Geschmack aufweisen, d. h. sie sind in der Lage, die Rezeptoren der Haarzellen des olfaktorischen Systems zu erregen. Osmophore Gruppen sind kovalent an das Molekülgerüst gebundene Gruppen in Form von Hydroxygruppen, Formylgruppen, Oxogruppen, Alkoxycarbonylgruppen, Nitrilgruppen, Nitrogruppen, Azidgruppen etc. In diesem Zusammenhang fallen unter den Begriff "Riechstoffe" im Sinne der vorliegenden Erfindung auch bei 20 °C und 1.013 hPa flüssige Parfümöle, Parfüme, oder Parfümölbestandteile.

Darüber hinaus werden unter dem Begriff "Wachse" im Rahmen der vorliegenden Erfindung Substanzen verstanden, welche bei 20 °C knetbar oder fest bis brüchig hart sind, eine grobe bis feinkristalline Struktur aufweisen und farblich durchscheinend bis opak, aber nicht glasartig sind. Weiterhin schmelzen diese Substanzen über 25 °C ohne Zersetzung, sind wenig oberhalb des Schmelzpunktes leicht flüssig (wenig viskos), weisen eine stark temperaturabhängige Konsistenz und Löslichkeit auf und sind unter leichtem Druck polierbar.

Der Begriff "flüchtiges kosmetisches Öl" bezeichnet erfindungsgemäß kosmetische Öle, welche bei 20 °C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige kosmetische Öle" im Sinne der vorliegenden Erfindung kosmetische Öle verstanden, welche bei 20 °C und einem Umgebungsdruck von 1.013 hPa einen Dampfdruck von weniger als 2,66 Pa (0,02 mm Hg) aufweisen.

Weiterhin sind unter dem Begriff der "Fettsäure", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff des "Fettalkohols" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nichts anderes angegeben ist, auf das Gesamtgewicht der erfindungsgemäßen schweißhemmenden kosmetischen Mittel ohne gegebenenfalls vorhandenes Treibmittel.

Gemäß einer bevorzugten Ausführungsform der vorliegenden Erfindung ist das bei 20°C und 1.013 hPa flüssige kosmetische Öl ausgewählt aus der Gruppe von
(i) flüchtigen cyclischen Siliconölen, insbesondere Cyclotrisiloxan, Cyclotetrasiloxan, Cyclopentasiloxan und Cyclohexasiloxan, und linearen Siliconölen mit 2 bis 10 Siloxaneinheiten, insbesondere Hexamethyldisiloxan, Octamethyltrisiloxan, Decamethyltetrasiloxan;
(ii) flüchtigen Nichtsiliconölen, insbesondere flüssigen Paraffinölen und Isoparaffinölen, wie Isodecan, Isoundecan, Isododecan, Isotridecan, Isotetradecan, Isopentadecan, Isohexadecan und Isoeicosan;
(iii) nichtflüchtigen Siliconölen, insbesondere höhermolekularen linearen Polyalkylsiloxane;
(iv) nichtflüchtigen Nichtsiliconölen, insbesondere den Estern von linearen oder verzweigten gesättigten oder ungesättigten C₂₋₃₀-Fettalkoholen mit linearen oder verzweigten gesättigten oder ungesättigten C₂₋₃₀-Fettsäuren, welche hydroxyliert sein können, den C₈-C₂₂-Fettalkoholestern einwertiger oder mehrwertiger C₂-C₇-Hydroxycarbonsäuren, den Triethylcitraten, den verzweigten gesättigten oder ungesättigten C₆₋₃₀-Fettalkoholen, den Mono-, Di- und Triglyceriden von linearen oder verzweigten, gesättigten oder ungesättigten, gegebenenfalls hydroxylierten C₈-₃₀-Fettsäuren, den Dicarbonsäureestern von linearen oder verzweigten C₂-C₁₀-Alkanolen, den Anlagerungsprodukten von Ethylenoxid und/oder Propylenoxid an ein- oder mehrwertige C₃₋₂₂-Alkanole, welche gegebenenfalls verestert sein können, den symmetrischen, unsymmetrischen oder cyclischen Estern der Kohlensäure mit Fettalkoholen, den Estern von Dimeren ungesättigter C₁₂₋₂₂-Fettsäuren mit einwertigen, linearen, verzweigten und cyclischen C₂₋₁₈-Alkanolen oder C₂₋₆-Alkanolen, den Benzoesäureestern von linearen oder verzweigten C₈₋₂₂-Alkanolen, wie Benzoesäure-C₁₂₋₁₅-Alkylester und Benzoesäureisostearylester und Benzoesäureoctyldodecylester, den synthetischen Kohlenwasserstoffen, wie Polyisobuten und Polydecene, den alicyclischen Kohlenwasserstoffen; sowie
(v) deren Mischungen.

Der Einsatz von flüchtigen Siliconölen und flüchtigen Nichtsiliconölen in den schweißhemmenden kosmetischen Mitteln resultiert in einem trockeneren Hautgefühl und in einer schnelleren Freisetzung des schweißhemmenden Aluminiumsalzes.

Die im Rahmen der Erfindung einsetzbaren cyclischen flüchtigen Siliconöle weisen bei 20 °C und einem Umgebungsdruck von 1.013 hPa einen Dampfdruck von 13 bis 15 Pa (0,1 mm Hg) auf. Weiterhin kann erfindungsgemäß als lineares flüchtiges Siliconöl auch ein niedermolekulares Phenyl Trimethicone mit einem Dampfdruck von etwa 2.000 Pa (15 mm Hg) bei 20 °C und einem Umgebungsdruck von 1.013 hPa eingesetzt werden. Aufgrund der hohen Persistenz von Cyclodimethiconen in der Umwelt kann es erfindungsgemäß jedoch bevorzugt sein, wenn in den schweißhemmenden kosmetischen Mitteln 0 bis weniger als 1 Gew.-%, vorzugsweise 0 bis weniger als 0,1 Gew.-%, cyclische flüchtige Siliconöle eingesetzt werden.

Erfindungsgemäß werden bevorzugt flüchtige Nichtsiliconöle in Form von C₁₀₋₁₃-Isoparaffin-Mischungen mit einem Dampfdruck von 10 bis 400 Pa (0,08 bis 3 mm Hg), vorzugsweise von 13 bis 100 Pa (0,1 bis 0,8 mm Hg), bei 20 °C und einem Umgebungsdruck von 1.013 hPa eingesetzt. Dabei ist es im Rahmen der vorliegenden Erfindung bevorzugt, wenn das flüchtige C₈-C₁₆-Isoparaffin in einer Gesamtmenge von 1 bis 60 Gew.-%, vorzugsweise von 3 bis 45 Gew.-%, bevorzugt von 5 bis 40 Gew.-%, insbesondere von 8 bis 20 Gew.-%, bezogen auf das Gesamtgewicht des schweißhemmenden kosmetischen Mittels, enthalten ist. Selbstverständlich ist es ebenfalls möglich, schweißhemmende kosmetische Mittel mit einem geringen Anteil an flüchtigen Ölen - das heißt, mit 0,5 bis 15 Gew.-%, bezogen auf das Gesamtgewicht des schweißhemmenden kosmetischen Mittels, an flüchtigen Ölen - oder sogar ohne flüchtige Öle zu formulieren.

Zur Maskierung von unlöslichen Bestandteilen, wie Talkum oder auf der Haut angetrockneten schweißhemmenden Aluminiumsalzen, kann es erfindungsgemäß bevorzugt sein, wenn die schweißhemmenden kosmetischen Mittel ein nichtflüchtiges Siliconöl und/oder ein nichtflüchtiges Nichtsiliconöl enthalten.

In diesem Zusammenhang enthalten bevorzugte schweißhemmende kosmetische Mittel mindestens einen Ester der linearen oder verzweigten gesättigten oder ungesättigten Fettalkohole mit 2 bis 30 Kohlenstoffatomen mit linearen oder verzweigten gesättigten oder ungesättigten Fettsäuren mit 2 bis 30 Kohlenstoffatomen, welche hydroxyliert sein können, in einer Gesamtmenge von 1 bis 30 Gew.-%, vorzugsweise von 5 bis 26 Gew.-%, bevorzugt von 9 bis 24 Gew.-%, insbesondere von 12 bis 17 Gew.-%, bezogen auf das Gesamtgewicht des schweißhemmenden kosmetischen Mittels.

Im Rahmen der vorliegenden Erfindung können als nichtflüchtige Siliconöle lineare Polyalkylsiloxane mit einer kinematischen Viskosität bei 25 °C von 5 bis 2.000 cSt, insbesondere linearen Polydimethylsiloxane mit einer kinematischen Viskosität bei 25 °C von 5 bis 2.000 cSt, vorzugsweise von 10 bis 350 cSt, insbesondere von 50 bis 100 cSt, eingesetzt werden. Die vorstehend genannten nichtflüchtigen Siliconöle sind unter den Handelsnamen Dow Corning® 200 bzw. Xiameter PMX von Dow Corning bzw. Xiameter erhältlich. Weitere bevorzugte nichtflüchtige Siliconöle sind Phenyltrimethicone mit einer kinematischen Viskosität bei 25 °C von 10 bis 100 cSt, vorzugsweise von 15 bis 30 cSt sowie Cetyldimethicone.

Erfindungsgemäß weiterhin bevorzugt ist der Einsatz von Mischungen der vorstehend genannten kosmetischen Öle, insbesondere von nichtflüchtigen und flüchtigen kosmetischen Ölen, da auf diese Weise Parameter wie Hautgefühl, Sichtbarkeit des Rückstands und Stabilität des schweißhemmenden kosmetischen Mittels eingestellt und das Mittel somit besser an die Bedürfnisse der Verbraucher angepasst werden kann.

Im Rahmen der vorliegenden Erfindung ist es bevorzugt, wenn das bei 20 °C und 1.013 hPa flüssige kosmetische Öl in einer Gesamtmenge von 1 bis 95 Gew.-%, vorzugsweise von 10 bis 85 Gew.-%, bevorzugt von 20 bis 75 Gew.-%, weiter bevorzugt von 35 bis 70 Gew.-%, insbesondere von 50 bis 60 Gew.-%, bezogen auf das Gesamtgewicht des schweißhemmenden kosmetischen Mittels, enthalten ist.

Es kann jedoch auch bevorzugt sein, wenn das bei 20 °C und 1.013 hPa flüssige kosmetische Öl in einer Gesamtmenge von 0,2 bis 70 Gew.-%, vorzugsweise von 2 bis 60 Gew.-%, bevorzugt von 3 bis 50 Gew.-%, weiter bevorzugt von 5 bis 35 Gew.-%, insbesondere von 8 bis 20 Gew.-%, bezogen auf das Gesamtgewicht des schweißhemmenden kosmetischen Mittels, enthalten ist. In diesem Zusammenhang kann es erfindungsgemäß auch vorgesehen sein, dass die schweißhemmenden Mittel weniger als 0,2 Gew.-%, vorzugsweise weniger als 0,1 Gew.-%, insbesondere 0 Gew.-% des bei 20 °C und 1.013 hPa flüssigen kosmetischen Öls enthalten. Der Einsatz nur äußerst geringer Mengen des bei 20 °C und 1.013 hPa flüssigen kosmetischen Öls ist insbesondere bei schweißhemmenden kosmetischen Mitteln bevorzugt, welche in wässriger, wässrig-alkoholischer oder wässrig-glykolischer Form vorliegen.

Gemäß einer bevorzugten Ausführungsform der vorliegenden Erfindung ist der Riechstoff ausgewählt aus der Gruppe von
(i) Estern, insbesondere Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenyl-glycinat, Allylcyclohexylpropionat, Styrallylpropionat, Benzylsalicylat, Cyclohexylsalicylat, Floramat, Melusat und Jasmecyclat;
(ii) Ethern, insbesondere Benzylethylether und Ambroxan;
(iii) Aldehyden, insbesondere linearen Alkanale mit 8 bis 18 Kohlenstoffatomen, Citral, Citronellal, Citronellyloxy-acetaldehyd, Cyclamenaldehyd, Lilial und Bourgeonal;
(iv) Ketonen, insbesondere Jonone, alpha-Isomethylionon und Methylcedrylketon;
(v) Alkoholen, insbesondere Anethol, Citronellol, Eugenol, Geraniol, Linalool, Phenylethylalkohol und Terpineol;
(vi) Kohlenwasserstoffen, insbesondere Terpene wie Limonen und Pinen; sowie
(vii) deren Mischungen.

Bevorzugt werden jedoch Mischungen verschiedener Riechstoffe verwendet, welche gemeinsam eine ansprechende Duftnote erzeugen.

Besonders ansprechend riechende schweißhemmende kosmetische Mittel werden erhalten, wenn der Riechstoff in einer Gesamtmenge von 0,00001 bis 10 Gew.-%, vorzugsweise von 0,001 bis 9 Gew.-%, bevorzugt von 0,01 bis 8 Gew.-%, weiter bevorzugt von 0,5 bis 7 Gew.-%, insbesondere von 1 bis 6 Gew.-%, bezogen auf das Gesamtgewicht des schweißhemmenden kosmetischen Mittels, enthalten ist. In diesem Zusammenhang kann es jedoch auch vorgesehen sein, dass der Riechstoff in einer Gesamtmenge von 0,00001 bis 5 Gew.-%, vorzugsweise 0,001 bis 4 Gew.-%, bevorzugt 0,01 bis 3 Gew.-%, weiter bevorzugt 0,1 bis 2 Gew.-%, insbesondere 0,2 bis 1,5 Gew.-%, bezogen auf das Gesamtgewicht des treibmittelhaltigen schweißhemmenden kosmetischen Mittels, enthalten ist.

Gemäß einer weiteren bevorzugten Ausführungsform der vorliegenden Erfindung ist das Wachs ausgewählt aus der Gruppe von
(i) Fettsäureglycerinmono-, -di- und -triestern;
(ii) Butyrospermum Parkii (Shea Butter);
(iii) Estern von gesättigten, einwertigen C₈₋₁₈-Alkoholen mit gesättigten C₁₂₋₁₈-Monocarbonsäuren;
(iv) linearen, primären C₁₂-C₂₄-Alkanolen;
(v) Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat;
(vi) Glycerintriestern von gesättigten linearen C₁₂-C₃₀-Carbonsäuren, die hydroxyliert sein können, insbesondere hydriertes Palmöl, hydriertes Kokosöl, hydriertes Rizinusöl, Glyceryltribehenat und Glyceryltri-12-hydroxystearat;
(vii) natürlichen pflanzlichen Wachsen, insbesondere Candelillawachs, Carnaubawachs, Japanwachs, Zuckerrohrwachs, Ouricourywachs, Korkwachs, Sonnenblumenwachs, Fruchtwachse;
(viii) tierischen Wachsen, insbesondere Bienenwachs, Schellackwachs und Walrat;
(ix) synthetischen Wachsen, insbesondere Montanesterwachse, hydrierte Jojobawachse und Sasolwachse, Polyalkylenwachse und Polyethylenglycolwachse, C₂₀-C₄₀-Dialkylester von Dimersäuren, C₃₀-₅₀-Alkylbienenwachs sowie Alkyl- und Alkylarylester von Dimerfettsäuren, Paraffinwachse; sowie
(x) deren Mischungen.

Besonders bevorzugt sind Handelsprodukte mit der INCI-Bezeichnung Cocoglycerides, insbesondere die Handelsprodukte Novata® (ex BASF), besonders bevorzugt Novata® AB, ein Gemisch aus C₁₂₋₁₈-Mono-, Di- und Triglyceriden, das im Bereich von 30 bis 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂₋₁₈-Alkoholen mit gesättigten C₁₂₋₁₈-Monocarbonsäuren sind Stearyllaurat, Cetearylstearat (z. B.

Crodamol® CSS), Cetylpalmitat (z. B. Cutina® CP) und Myristylmyristat (z. B. Cetiol® MM). Weiterhin wird bevorzugt ein C₂₀-C₄₀-Alkylstearat als Wachskomponente eingesetzt. Dieser Ester ist unter den Namen Kesterwachs® K82H oder Kesterwachs® K80H bekannt und wird von Koster Keunen Inc. vertrieben.

Im Rahmen der vorliegenden Erfindung ist es bevorzugt, wenn das Wachs in einer Gesamtmenge von 0,01 bis 20 Gew.-%, vorzugsweise von 3 bis 20 Gew.-%, bevorzugt von 5 bis 18 Gew.-%, insbesondere von 6 bis 15 Gew.-%, bezogen auf das Gesamtgewicht des schweißhemmenden kosmetischen Mittels, enthalten ist.

Eine besonders gute schweißhemmende Wirkung wird im Rahmen der vorliegenden Erfindung erhalten, wenn das schweißhemmende Aluminiumsalz in einer Gesamtmenge von 2 bis 40 Gew.-%, vorzugsweise von 3 bis 35 Gew.-%, bevorzugt von 4 bis 32 Gew.-%, weiter bevorzugt von 5 bis 28 Gew.-%, noch mehr bevorzugt von 8 bis 25 Gew.-%, insbesondere von 12 bis 22 Gew.-%, bezogen auf das Gesamtgewicht des schweißhemmenden kosmetischen Mittels, enthalten ist. Es kann jedoch auch vorgesehen sein, dass das schweißhemmende Aluminiumsalz in einer Gesamtmenge von 0,1 bis 20 Gew.-%, vorzugsweise von 0,5 bis 15 Gew.-%, bevorzugt von 1 bis 12 Gew.-%, weiter bevorzugt von 1,5 bis 10 Gew.-%, insbesondere von 2 bis 8 Gew.-%, bezogen auf das Gesamtgewicht der treibmittelhaltigen schweißhemmenden Zusammensetzung, enthalten ist.

Im Rahmen der vorliegenden Erfindung kann das schweißhemmende Aluminiumsalz ausgewählt sein aus der Gruppe von
(i) wasserlöslichen adstringierenden anorganischen Salzen des Aluminiums, insbesondere Aluminiumchlorhydrat, Aluminiumsesquichlorohydrat, Aluminiumdichlorohydrat, Aluminium-hydroxid, Kaliumaluminiumsulfat, Aluminiumbromhydrat, Aluminiumchlorid, Aluminiumsulfat;
(ii) wasserlöslichen adstringierenden organischen Salzen des Aluminiums, insbesondere Aluminiumchlorhydrex-Propylenglycol, Aluminiumchlorhydrex-Polyethylenglycol, Aluminium-Propylenglycol-Komplexe, Aluminiumsesquichlorhydrex-Propylenglycol, Aluminiumsesqui-chlorhydrex-Polyethylenglycol, Aluminium-Propylenglycol-dichlorhydrex, Aluminium-Poly-ethylenglycoldichlorhydrex, Aluminiumundecylenoylcollagenaminosäure, Natriumaluminium-lactat, Natriumaluminiumchlorhydroxylactat, Aluminium-lipoaminosäuren, Aluminiumlactat, Aluminiumchlorohydroxyallantoinat, Natrium-Aluminium-Chlorohydroxylactat;
(iii) wasserlöslichen adstringierenden anorganischen Aluminium-Zirkonium-Salzen, insbesondere Aluminiumzirconiumtrichlorhydrat, Aluminiumzirconiumtetrachlorhydrat, Aluminiumzirconiumpentachlorhydrat, Aluminiumzirconiumoctachlorhydrat;
(iv) wasserlöslichen adstringierenden organischen Aluminium-Zirkonium-Salzen, insbesondere Aluminiumzirkonium-Propylenglycol-Komplexe, Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin; sowie
(v) deren Mischungen.

Unter dem Begriff "schweißhemmende Aluminiumsalze" werden erfindungsgemäß keine Alumosilicate und Zeolithe verstanden. Weiterhin werden erfindungsgemäß unter wasserlöslichen Aluminiumsalzen solche Salze verstanden, welche eine Löslichkeit von mindestens 3 Gew.-% bei 20 °C aufweisen, d. h. es lösen sich mindestens 3 g des schweißhemmenden Aluminiumsalzes in 97 g Wasser bei 20 °C.

Besonders bevorzugte anorganische Aluminiumsalze sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl. 2-3 H₂O]ₙ, das in nichtaktivierter (polymerisierter) oder in aktivierter (depolymerisierter) Form vorliegen kann, sowie Aluminiumchlorhydrat mit der allgemeinen Formel [Al₂(OH)₄Cl₂· 1-6 H₂O]ₙ, bevorzugt [Al₂(OH)₄Cl₂ · 2-3 H₂O]ₙ, das in nichtaktivierter (polymerisierter) oder in aktivierter (depolymerisierter) Form vorliegen kann. Die Herstellung derartiger schweißhemmender Aluminiumsalze ist beispielsweise in den Druckschriften US 3 887 692 A, US 3 904 741 A, US 4 359 456 A, GB 2 048 229 A und GB 1 347 950 A offenbart.

Erfindungsgemäß besonders bevorzugte schweißhemmende Aluminiumsalze sind ausgewählt aus sogenannten "aktivierten" Aluminiumsalzen, welche auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in den Druckschriften GB 2 048 229 A, US 4 775 528 A und US 6 010 688 A offenbart. Aktivierte Aluminiumsalze werden in der Regel durch Wärmebehandlung einer verdünnten Lösung des entsprechenden Salzes erzeugt (z.B. einer Lösung mit 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet, werden. Neben der Sprühtrocknung ist z. B. auch die Walzentrocknung geeignet. Aktivierte Aluminiumsalze haben typischerweise ein HPLC-Peak 4-zu-Peak 3-Flächenverhältnis von mindestens 0,4, vorzugsweise von mindestens 0,7, insbesondere von mindestens 0,9, wobei mindestens 70% des Aluminiums diesen HPLC-Peaks zuzuordnen sind.

In diesem Zusammenhang sind ebenfalls "aktiviertes" Aluminium-Zirkoniumsalze bekannt, welche einen hohen HPLC-Peak 5-Aluminium-Gehalt, insbesondere eine Peak 5-Fläche von mindestens 33 %, vorzugsweise von mindestens 45 %, bezogen auf die gesamte Fläche unter den Peaks 2 bis 5, gemessen mit HPLC einer 10 Gew.-%igen wässrigen Lösung des Wirkstoffs unter Bedingungen, bei welchen die Aluminiumspecies in mindestens 4 aufeinander folgende Peaks aufgelöst werden (mit Peaks 2 bis 5 bezeichnet) aufweisen. Bevorzugte Aluminium-Zirconiumsalze mit einem hohen HPLC-Peak 5-Aluminium-Gehalt (auch als "E⁵AZCH" bezeichnet) sind beispielsweise in den Druckschriften US 6 436 381 A und US 6 649 152 A offenbart. Weiterhin können die vorstehend genannten aktivierten Aluminium-Zirkoniumsalz zusätzlich mit einem wasserlöslichen Strontiumsalz und/oder mit einem wasserlöslichen Calciumsalz stabilisiert sein, wie sie beispielsweise in der Druckschrift US 6 923 952 A offenbart sind.

Es ist erfindungsgemäß ebenfalls möglich, schweißhemmende Aluminiumsalze als nichtwässrige Lösungen oder Solubilisate eines aktivierten schweißhemmenden Aluminium- oder Aluminium-Zirkoniumsalzes, beispielsweise gemäß der Druckschrift US 6 010 688 A, einzusetzen. Derartige Aluminium- bzw. Aluminium-Zirkoniumsalze werden durch den Zusatz einer wirksamen Menge eines mehrwertigen Alkohols, welcher 3 bis 6 Kohlenstoffatome und 3 bis 6 Hydroxylgruppen, bevorzugt Propylenglycol, Sorbit und Pentaerythrit, aufweist, gegen den Verlust der Aktivierung des Salzes stabilisiert.

Besonders bevorzugt sind auch Komplexe aktivierter schweißhemmender Aluminium- bzw. Aluminium-Zirkoniumsalze mit einem mehrwertigen Alkohol, welche 20 bis 50 Gew.-%, vorzugsweise 20 bis 42 Gew.-%, aktiviertes schweißhemmendes Aluminium- bzw. Aluminium-Zirkoniumsalz und 2 bis 16 Gew.-% molekular gebundenes Wasser enthalten, wobei der Rest zu 100 Gew.-% mindestens ein mehrwertiger Alkohol mit 3 bis 6 Kohlenstoffatomen und 3 bis 6 Hydroxylgruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- bzw. Aluminium-Zirkoniumsalzes mit einem mehrwertigen Alkohol sind z. B. in den Druckschriften US 5 643 558 A und US 6 245 325 A offenbart.

Im Rahmen der vorliegenden Erfindung ist es ebenfalls möglich, als schweißhemmende Aluminiumsalze basische Calcium-Aluminiumsalze, wie sie z. B. in der Druckschrift US 2 571 030 A offenbart sind, einzusetzen. Diese Salze können durch Umsetzung von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid erhalten werden. Es ist jedoch ebenfalls möglich, Aluminium-Zirconium-Komplexe, welche mit Salzen von Aminosäuren, insbesondere mit Alkali- und Erdalkaliglycinaten, gepuffert sind und wie sie z. B. in der Druckschrift US 4 017 599 A offenbart sind, einzusetzen.

Als erfindungsgemäß bevorzugte schweißhemmende aktivierte Aluminium- und Aluminium-Zirkoniumsalze können auch die in den nachfolgenden Druckschriften US 6 245 325 A, US 6 042 816 A, US 6 245 325 A, US 6 042 816 A, US 6 245 325 A, US 6 042 816 A, US 6 245 325 A, US 6 042 816 A oder US 7 105 691 A angeführten Aluminium bzw. Aluminium-Zirkoniumsalze eingesetzt werden, welche bevorzugt durch Aminosäuren, insbesondere Glycin, Hydroxyalkansäuren, insbesondere Glycolsäure und Milchsäure, oder Betaine stabilisiert sind.

Weitere bevorzugte aktivierte Aluminiumsalze sind solche der allgemeinen Formel Al₂(OH)₆₋ₐXa, worin X für Cl, Br, I oder NO₃ und "a" für eine Zahl von 0,3 bis 5, vorzugsweise von 0,8 bis 2,5 insbesondere von 1 bis 2 steht, so dass das Molverhältnis von AI:X 0,9:1 bis 2,1:1 beträgt. Derartige aktivierte schweißhemmende Aluminiumsalze sind beispielsweise in der Druckschrift US 6 074 632 A offenbart. Besonders bevorzugt ist Aluminiumchlorhydrat (d.h. X steht in der vorgenannten Formel für Cl) und speziell 5/6-basisches Aluminiumchlorhydrat mit "a" = 1, so dass das Molverhältnis von Aluminium zu Chlor 1,9 : 1 bis 2,1 : 1 beträgt.

Bevorzugte aktivierte Aluminium-Zirconiumsalze sind solche der allgemeinen Formel ZrO(OH)₂-_{pb}Y_{b}, worin Y für Cl, Br, I, NO₃ oder SO₄, b für eine rationale Zahl von 0,8 bis 2 und p für die Wertigkeit von Y steht, so dass das Al:Zr-Molverhältnis von 2 bis 10 und das Metall:(X+Y)-Verhältnis von 0,73 bis 2,1, vorzugsweise von 0,9 bis 1,5 beträgt. Derartige aktivierte schweißhemmende Aluminium-Zirkoniumsalze sind beispielsweise in der zuvor genannten Druckschrift US 6 074 632 A offenbart. Ein besonders bevorzugtes Salz ist Aluminium-Zirconiumchlorhydrat (d.h. X und Y stehen für Cl), welches ein Al:Zr-Verhältnis von 2 bis 10 und ein molares Metall:Cl-Verhältnis von 0,9 bis 2,1 aufweist. Bevorzugte schweißhemmende Wirkstoffe sind in den Druckschriften US 6 663 854 A und US 2004/0009133 A1 offenbart.

Erfindungsgemäß besonders bevorzugte schweißhemmende Aluminiumsalze weisen ein molares Metall-zu-Chlorid-Verhältnis von 1,9 bis 2,1 auf. Das Metall-zu-Chlorid-Verhältnis von im Rahmen der Erfindung ebenfalls besonders bevorzugten Aluminiumsesquichlorohydraten beträgt 1,5 : 1 bis 1,8 : 1. Bevorzugte Aluminium-Zirconiumtetrachlorohydrate weisen ein molares Verhältnis von AI:Zr von 2 bis 6 und von Metall : Chlorid von 0.9 bis 1.3 auf, wobei insbesondere Salze mit einem molaren Metall-zu-Chlorid-Verhältnis von 0,9 bis 1,1, vorzugsweise von 0,9 bis 1,0 bevorzugt sind.

Die Kombination bzw. Mischung der Methansulfonsäure bzw. deren Salze (Formel S-I) mit einem schweißhemmenden Aluminiumsalz resultiert in einer erhöhten Aktivierung der schweißhemmenden Aluminiumsalze und führt darüber hinaus zu einer besonders effektiven Stabilisierung der gebildeten kurzkettigen Polymere, so dass die erfindungsgemäßen schweißhemmenden kosmetischen Mittel eine signifikant verbesserte Antitranspirantwirkung aufweisen.

Gemäß einer weiteren Ausführungsform der vorliegenden Erfindung enthält das schweißhemmende kosmetische Mittel keine zirkoniumhaltigen Verbindungen. Die Vermeidung des Einsatzes von zirkoniumhaltigen schweißhemmenden Verbindungen, wie Zirkonium-Aluminium-Mischsalzen resultiert in der kostengünstigeren Bereitstellung der schweißhemmenden kosmetischen Mittel, da die Rohstoffe zur Herstellung der zirkoniumhaltigen Verbindungen höhere Preise aufweisen.

Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des schweißhemmenden kosmetischen Mittels. Unter freiem Wasser im Sinne der vorliegenden Erfindung wird somit Wasser verstanden, welches von Kristallwasser, Hydratationswasser oder ähnlich molekular gebundenem Wasser der eingesetzten Bestandteile, insbesondere der schweißhemmenden Aluminiumsalze, verschieden ist.

Überraschenderweise wurde festgestellt, dass die Menge an kurzkettigen Polymeren bei Kombination eines schweißhemmenden Aluminiumsalzes mit einer Verbindung der Formel (S-I) signifikant gesteigert werden kann, wenn die schweißhemmenden kosmetischen Mittel freies Wasser in einer Menge von 15 bis 96 Gew.-%, bezogen auf das Gesamtgewicht des schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 15 bis 96 Gew.-%, vorzugsweise von 25 bis 80 Gew.-%, bevorzugt von 30 bis 70 Gew.-%, insbesondere von 40 bis 60 Gew.-%, bezogen auf das Gesamtgewicht des schweißhemmenden kosmetischen Mittels.

Die schweißhemmenden kosmetischen Mittel liegen bevorzugt als Suspension des ungelösten schweißhemmenden Aluminiumsalzes in dem bei 20 °C und 1.013 hPa flüssigen kosmetischen Öl vor.

In einer weiteren bevorzugten Darreichungsform liegt das schweißhemmende kosmetische Mittel als Wasser-in-Öl-Emulsion vor. Hierbei kann es sich insbesondere um eine versprühbare Wasser-in-Öl-Emulsion handeln, welche mittels eines Treibmittels versprüht werden kann. In diesem Zusammenhang ist es bevorzugt, wenn das in Form einer Wasser-in-ÖI-Emulsion vorliegende schweißhemmende kosmetische Mittel die Verbindung der Formel (S-I) in einer Gesamtmenge von 0,05 bis 8 Gew.-%, vorzugsweise von 0,1 bis 7 Gew.-%, bevorzugt von 0,3 bis 5 Gew.-%, weiter bevorzugt von 0,5 bis 3 Gew.-%, noch weiter bevorzugt von 0,8 bis 2,5 Gew.-%, am insbesondere von 1 bis 2 Gew.-%, bezogen auf das Gesamtgewicht des schweißhemmenden kosmetischen Mittels, enthält.

Im Rahmen der vorliegenden Erfindung kann es auch vorgesehen sein, das schweißhemmende kosmetische Mittel als Öl-in-Wasser-Emulsion vorliegt. In diesem Fall wird das Mittel bevorzugt als treibmittelfreies Pumpspray oder Quetschspray versprüht oder als Roll-on appliziert. In diesem Zusammenhang ist es bevorzugt, wenn das in Form einer Öl-in-Wasser-Emulsion vorliegende schweißhemmende kosmetische Mittel die Verbindung der Formel (S-I) in einer Gesamtmenge von 0,05 bis 8 Gew.-%, vorzugsweise von 0,1 bis 7 Gew.-%, bevorzugt von 0,3 bis 5 Gew.-%, weiter bevorzugt von 0,5 bis 3 Gew.-%, noch weiter bevorzugt von 0,8 bis 2,5 Gew.-%, insbesondere von 1 bis 2 Gew.-%, bezogen auf das Gesamtgewicht des schweißhemmenden kosmetischen Mittels, enthält.

Erfindungsgemäß kann es weiterhin vorgesehen sein, dass das schweißhemmende kosmetische Mittel als wässrige, wässrig-alkoholische oder wässrig-glykolische Lösung vorliegt. Aufgrund der erfindungsgemäßen Kombination eines schweißhemmenden Aluminiumsalzes mit einer Verbindung der Formel (S-I) können selbst in protischen Lösungsmitteln, wie wässrigen Lösungen, hohe Mengen an aktiviertem Aluminiumsalz gebildet bzw. stabilisiert werden, so dass erfindungsgemäß auch der Einsatz von wässrigen schweißhemmenden kosmetischen Mitteln möglich ist, ohne dass eine Desaktivierung der schweißhemmenden Aluminiumsalze und damit eine signifikante Verringerung der Antitranspirantwirkung auftritt.

Gemäß einer bevorzugten Ausführungsform der vorliegenden Erfindung enthält das schweißhemmende kosmetische Mittel Ethanol in einer Gesamtmenge von 1 bis 50 Gew.-%, vorzugsweise von 5 bis 40 Gew.-%, bevorzugt von 7 bis 35 Gew.-%, insbesondere von 10 bis 30 Gew.-%, bezogen auf das Gesamtgewicht des schweißhemmenden kosmetischen Mittels. Es kann jedoch auch vorgesehen sein, dass das schweißhemmenden kosmetische Mittel Ethanol in einer Gesamtmenge von 10 bis 95 Gew.-%, vorzugsweise von 15 bis 90 Gew.-%, bevorzugt von 20 bis 87 Gew.-%, weiter bevorzugt von 30 bis 85 Gew.-%, insbesondere von 40 bis 80 Gew.-%, bezogen auf das Gesamtgewicht des schweißhemmenden kosmetischen Mittels, enthält. Wie zuvor ausgeführt, können durch den Einsatz der Verbindung der Formel (S-I) selbst hohe Mengen an protischen Lösungsmitteln, wie Ethanol, verwendet werden, ohne dass die Aktivierung bzw. die Stabilisierung der kurzkettigen Polymere des schweißhemmenden Aluminiumsalzes negativ beeinflusst wird.

Die Applikation des schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Behälter weisen eine Entnahmevorrichtung auf, bevorzugt in Gestalt von Ventilen, welche die Entnahme des Inhalts als Nebel, Rauch, Schaum, Pulver, Paste oder Flüssigkeitsstrahl ermöglichen. Als Behälter für die Sprühvorrichtungen kommen vor allem zylindrische Gefäße aus Metall (Aluminium, Weißblech, Rauminhalt bevorzugt maximal 1.000 ml), geschütztem bzw. nichtsplitterndem Glas oder Kunststoff (Rauminhalt bevorzugt maximal 220 ml) bzw. splitterndem Glas oder Kunststoff (Rauminhalt bevorzugt 50 bis 400 ml) in Frage. Cremeförmige, gelförmige, pastöse und flüssige Mittel können z.B. in Pump-, Spray- oder Quetschspendern abgepackt sein, insbesondere auch in Mehrkammer-Pump-, Mehrkammer-Spray- oder Mehrkammer-Quetschspendern. Die Verpackung für die Mittel kann undurchsichtig, aber auch transparent oder transluzent sein.

Das schweißhemmende kosmetische Mittel ist bevorzugt als Stift, Soft Solid, Creme, Roll-on, Dibenzylidenalditol-basiertes Gel, loses oder kompaktes Puder konfektioniert. Die Formulierung der schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können.

Die Applikation kann beispielsweise mit einem Rollkugelapplikator erfolgen. Solche Roller weisen eine in einem Kugelbett gelagerte Kugel auf, die durch Bewegung über eine Oberfläche bewegt werden kann. Dabei nimmt die Kugel etwas von dem zu verteilenden schweißhemmenden Mittel auf und befördert dieses an die zu behandelnde Oberfläche. Die Verpackung für die erfindungsgemäßen Mittel kann, wie zuvor ausgeführt, undurchsichtig, transparent oder transluzent sein.

Weiterhin ist es auch möglich, die schweißhemmenden kosmetischen Mittel mittels eines festen Stifts zu applizieren.

Es kann erfindungsgemäß jedoch auch bevorzugt sein, dass das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem schweißhemmenden kosmetischen Mittel imprägniert oder beaufschlagt und bevorzugt einzeln verpackt. Sie können z. B. als Deodorant-Tuch eingesetzt werden, was besonders interessant für den Gebrauch unterwegs ist. Bevorzugte Substratmaterialien sind ausgewählt aus porösen flächigen Tüchern. Sie können aus einem faserigen oder zellulären flexiblen Material bestehen, das ausreichend mechanische Stabilität und gleichzeitig Weichheit zur Anwendung auf der Haut aufweist. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines schweißhemmenden kosmetischen Mittels auf ein Substrat erhalten werden.

Die schweißhemmenden kosmetischen Mittel können darüber hinaus weitere Hilfsstoffe enthalten. Bevorzugt enthalten die schweißhemmenden kosmetischen Mittel mindestens einen weiten Hilfsstoff, ausgewählt aus der Gruppe von (i) Emulgatoren und/oder Tensiden; (ii) Hydrogelbildnern; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) Treibmitteln; (viii) Verdickungsmitteln sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear.

Unter anionischen Tensiden werden Tenside mit ausschließlich anionischen Ladungen verstanden; sie enthalten z. B. Carboxylgruppen, Sulfonsäuregruppen oder Sulfatgruppen. Besonders bevorzugte anionische Tenside sind Alkylsulfate, Alkylethersulfate, Acylglutamate und C₈₋₂₄-Carbonsäuren sowie deren Salze, die sogenannten Seifen.

Unter kationischen Tensiden werden Tenside mit ausschließlich kationischen Ladungen verstanden; sie enthalten z. B. quartäre Ammoniumgruppen. Bevorzugt sind kationische Tenside vom Typ der quartären Ammoniumverbindungen, der Esterquats und der Amidoamine. Bevorzugte quaternäre Ammoniumverbindungen sind Ammoniumhalogenide sowie die unter den INCI-Bezeich-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder -SO₃H-Gruppen) als auch basische hydrophile Gruppen (beispielsweise Aminogruppen) besitzen und sich also je nach Bedingung sauer oder basisch verhalten. Unter zwitterionischen Tensiden versteht der Fachmann Tenside, die im selben Molekül sowohl eine negative als auch eine positive Ladung tragen. Beispiele für bevorzugte zwitterionische Tenside sind die Betaine, die N-Alkyl-N,N-dimethylammonium-glycinate, die N-Acyl-aminopropyl-N,N-dimethylammoniumglycinate und die 2-Alkyl-3-carboxymethyl-3-hydroxyethyl-imidazoline mit jeweils 8 bis 24 Kohlenstoffatomen in der Alkylgruppe. Beispiele für bevorzugte ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropyl-glycine, N-Alkyltaurine, N-Alkylsarcosine, 2-Alkylaminopropionsäuren und Alkylaminoessigsäuren mit jeweils 8 bis 24 Kohlenstoffatomen in der Alkylgruppe

Die Zusammensetzungen, welche als Emulsion, insbesondere als Öl-in-Wasser-Emulsion, formuliert sind, enthalten bevorzugt mindestens einen nichtionischen Öl-in-Wasser-Emulgator mit einem HLB-Wert von mehr als 7 bis 20. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. In diesem Zusammenhang kann es erfindungsgemäß bevorzugt sein, wenn weiterhin ein Wasser-in-ÖI-Emulgator mit einem HLB-Wert von größer 1,0 und kleiner/gleich 7,0 eingesetzt wird. Im Rahmen der vorliegenden Erfindung geeignete nichtionische Öl-in-Wasser-Emulgatoren und geeignete nichtionische Wasser-in-Öl-Emulgatoren sind beispielsweise in der deutschen Offenlegungsschrift DE 10 2006 004 957 A1 beschrieben.

Zur Verdickung der schweißhemmenden kosmetischen Mittel werden bevorzugt Hydrogel bildende Substanzen eingesetzt, welche ausgewählt sind aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, weiterhin Polyvinylpyrrolidonen, Polyvinylalkoholen, Polyacrylamiden, weiterhin - wenn auch weniger bevorzugt - physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen, weiterhin - ebenfalls weniger bevorzugt - Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Hydrogelbildner sind ausgewählt aus Celluloseethern, vor allem aus Hydroxyalkylcellulosen, insbesondere aus Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon. Bevorzugt wird Hydroxyethylcellulose als Hydrogelbildner eingesetzt.

Um die aktivierende Wirkung der Verbindung der Formel (S-I) weiter zu unterstützen, kann es von Vorteil sein, den schweißhemmenden kosmetischen Mitteln mindestens einen Chelatbildner, der ausgewählt ist aus Ethylendiamintetraessigsäure (EDTA) und ihren Salze sowie aus Nitrilotriessigsäure (NTA) und Mischungen dieser Substanzen, in einer Gesamtmenge von 0,01 bis 0,5 Gew.-%, vorzugsweise von 0,02 bis 0,3 Gew.-%, insbesondere von 0,05 bis 0,1 Gew.-%, bezogen auf das Gesamtgewicht des schweißhemmenden Mittels, zuzusetzen. Im Rahmen der vorliegenden Erfindung können jedoch auch Chelatbildner eingesetzt werden, welche ausgewählt sind aus der Gruppe von β-Alanindiessigsäure, Cyclodextrin, Diethylentriaminpentamethylenphosphonsäure, Natrium-, Kalium-, Calciumdinatrium-, Ammonium- und Triethanolaminsalzen der Ethylendiamintetraessigsäure (EDTA), Etidronsäure, Hydroxyethylethylendiamintetraessigsäure (HEDTA) und ihren Natriumsalzen, Natriumsalzen der Nitrilotriessigsäure (NTA), Diethylentriaminpentaessigsäure, Phytinsäure, Hydroxypropylcyclodextrin, Methylcyclodextrin, Aminotrimethylen-phosphonat-Pentanatrium, Ethylendiamintetramethylenphosphonat-Pentanatrium, Diethylentriaminpentaacetat-Pentanatrium, Pentanatriumtriphosphat, Kalium-EDTMP, Natrium-EDTMP, Natriumdihydroxyethylglycinat, Natriumphytat, Natriumpolydimethylglycinophenolsulfonat, Tetrahydroxyethylethylendiamin, Tetrahydroxypropylethylendiamin, Tetrakaliumetidronat, Tetranatriumetidronat, Tetranatriumiminodisuccinat, Trinatriumethylendiamindisuccinat und Desferrioxamin.

Die Antitranspirantwirkung der schweißhemmenden kosmetischen Mittel kann weitergehend gesteigert werden, wenn mindestens einen Deodorant-Wirkstoff in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des schweißhemmenden kosmetischen Mittels, enthalten ist. Sofern Ethanol in den erfindungsgemäßen Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind ausgewählt aus der Gruppe der (i) Silbersalze; (ii) aromatischen Alkohole, insbesondere 2-Benzylheptan-1-ol sowie Mischungen von 2-Benzylheptan-1-ol und Phenoxyethanol; (iii) 1,2-Alkandiole mit 5 bis 12 Kohlenstoffatomen, insbesondere 3-(2-Ethylhexyloxy)-1,2-propandiol; (iv) Triethylcitrate; (v) Wirkstoffe gegen Exoesterasen, insbesondere gegen Arylsulfatase, Lipase, beta-Glucuronidase und Cystathion-β-lyase; (vi) kationischen Phospholipide; (vii) Geruchsabsorber, insbesondere Silicate, wie Montmorillonit, Kaolinit, Ilit, Beidellit, Nontronit, Saponit, Hectorit, Bentonit, Smectit und Talkum, Zeolithe, Zinkricinoleat, Cyclodextrine; (viii) desodorierend wirkenden Ionenaustauscher; (ix) keimhemmenden Mittel; (x) präbiotisch wirksamen Komponenten; sowie (xi) Mischungen dieser Deodorant-Wirkstoffe.

Bevorzugte schweißhemmenden kosmetische Mittel enthalten weiterhin mindestens ein wasserlösliches mehrwertiges C₂₋₉-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole sind ausgewählt aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Gemäß einer weiteren Ausführungsform der vorliegenden Erfindung enthalten die schweißhemmenden kosmetischen Mittel weiterhin mindestens einen hautkühlenden Wirkstoff.

Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Weiterhin kann es vorgesehen sein, dass die schweißhemmenden kosmetischen Mittel ein Treibmittel enthalten. In diesem Fall sind sie als treibgasgetriebenes Aerosol konfektioniert. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Gesamtmenge der Treibmittel beträgt 20 bis 95 Gew.-%, vorzugsweise 30 bis 85 Gew.-%, insbesondere 40 bis 75 Gew.-%, jeweils bezogen auf das Gesamtgewicht des Antitranspirants, bestehend aus dem schweißhemmenden kosmetischen Mittel und dem Treibmittel. In diesem Zusammenhang kann es auch vorgesehen sein, dass die Gesamtmenge an Treibmittel 1 bis 95 Gew.-%, vorzugsweise 2 bis 85 Gew.-%, insbesondere 3 bis 75 Gew.-%, jeweils bezogen auf das Gesamtgewicht des Antitranspirants, bestehend aus dem schweißhemmenden kosmetischen Mittel und dem Treibmittel, beträgt.

Als Hilfstoffe können erfindungsgemäß weiterhin lipophile Verdickungsmittel eingesetzt werden. Bevorzugt ist das mindestens eine schweißhemmende Aluminiumsalz ungelöst in mindestens einem bei 20 °C und 1.013 hPa flüssigen kosmetischen Öl suspendiert. Zur besseren Anwendbarkeit kann dieser Suspension noch mindestens ein lipophiles Verdickungsmittel als Suspendierhilfe zugesetzt werden. Erfindungsgemäß bevorzugte lipophile Verdickungsmittel sind ausgewählt aus hydrophobierten Tonmineralien und pyrogenen Kieselsäuren.

Die folgenden Beispiele erläutern die vorliegende Erfindung, ohne sie jedoch darauf einzuschränken:

### Beispiele:

### 1. HPLC-Messungen:

Zur Bestimmung der Aktivierung des schweißhemmenden Aluminiumsalzes in dem schweißhemmenden kosmetischen Mittel werden die folgenden wässrigen Lösungen hergestellt und für 2 bzw. 4 Wochen bei Raumtemperatur gelagert (Mengenangaben in Gewichtsprozent):
a) Stabilisierung bzw. Aktivierung eines bereits aktivierten Aluminiumchlorohydrat Pulvers in einer wässrigen Lösung (2 Wochen Lagerung bei Raumtemperatur)

| | V-I | E-I** | E-II** | E-III** |
|---|---|---|---|---|
| Reach 103, aktiviertes Aluminiumchlorohydrat (ACH) | 10* | 10* | 10* | 10* |
| Methansulfonsäure (S-Ia) | - | 0,5 | 1 | 2 |
| Wasser | 90 | 89,5 | 89 | 88 |

| | | | | |
|---|---|---|---|---|
| *Aktivsubstanz ** erfindungsgemäß | | | | |

b) Aktivierung eines nicht aktivierten Aluminiumchlorohydrats in einer wässrigen Lösung (4 Wochen Lagerung bei Raumtemperatur)

| | V-II | E-III** |
|---|---|---|
| Nicht aktiviertes Aluminiumchlorohydrat (ACH) | 10* | 10* |
| Methansulfonsäure (S-Ia) | - | 2,0 |
| Wasser | 90 | 88 |

| | | |
|---|---|---|
| *Aktivsubstanz ** erfindungsgemäß | | |

Derartige Lösungen sind unter anderem repräsentativ für wasserhaltige Antitranspirant-Emulsionen (Antitranspirant-Rollons; Antitranspirant-Stifte, Antitranspirant-Gele, Antitranspirant-Pumpzerstäuber).

Nach 2 bzw. 4 Wochen wird die Aktivierung bzw. Stabilisierung, welche sich unmittelbar aus der Polymerverteilung der Proben V-I, V-II, E-I, E-II und E-III ergibt, mittels Größenausschlusschromatographie (auch als "size exclusion chromatography" oder SEC bezeichnet) bestimmt. Hierzu wird die jeweilige Probe zunächst auf die Konzentration 1 g ACH / 25 ml Wasser verdünnt und filtriert (0,2 µm Filter). Anschließend wird die jeweilige Probe unter Verwendung einer handelsüblichen HPLC-Anlage mit einem Brechungsindexdetektor mittels SEC vermessen, wobei die folgenden Parameter verwendet wurden:

| | |
|---|---|
| Fluss: | 1 ml/min |
| Temperatur: | 23 °C |
| Eluent: | 0,02M HCl |
| Säule: | 6,2 x 250 mm, Säulenmaterial: silanisierte poröse Silica-Mikrospheren (5 µm). |

Für jede Probe werden 11 bzw. 12 oder mehr Peaks erhalten, wobei die Peaks mit der geringeren Retentionszeit für die langkettigen Polymere des ACH stehen, die Peaks mit höheren Retentionszeiten für Polymere des ACH mit kürzeren Kettenlängen. Die für jede Probe im Chromatogramm erhaltenen Peaks werden integriert, wobei die Peaks 2 und 3 (langkettige Polymere), 4 bis 8 (Polymere mit mittlerer Kettenlänge), 9 bis 11 (Polymere mit kurzer Kettenlänge) und 12 sowie alle nachfolgenden Peaks (sehr kurze Kettenlänge der Polymere) aufgrund von Schultern gemeinsam integriert werden.

In der nachfolgenden Tabelle ist der Flächenanteil der Peaks 1, 2 bis 3, 4 bis 8, 9 bis 11 und 12 und mehr an der Gesamtfläche aller für die jeweilige Probe im Chromatogramm erhaltenen Peaks angegeben, welche für die Aktivierung bzw. Stabilisierung eines bereits aktivierten Aluminiumchlorohydrat Pulvers erhalten wurden:

| | Peak 1 | Peak 2 - 3 | Peak 4 - 8 | Peak 9 - 11 | Peak 12 und > 12 |
|---|---|---|---|---|---|
| V-I | 0 | 8 | 56 | 31 | 5 |
| E-I | 0 | 5 | 49 | 38 | 9 |
| E-II | 0 | 4 | 46 | 38 | 12 |
| E-III | 0 | 4 | 41 | 41 | 14 |

Die erfindungsgemäßen Rezepturen E-I, E-II und E-III weisen einen signifikant höheren Anteil an kurzkettigen Polymeren (Peaks 9 bis 12 und mehr), d. h. eine deutlich bessere Aktivierung und Stabilisierung, auf als das Vergleichsbeispiel V-I. Weiterhin ist aus der obigen Tabelle ersichtlich, dass die Polymerverteilung durch die Erhöhung der Menge an Methansulfonsäure zugunsten der kurzkettigen Polymere verschoben werden kann. Folglich resultiert eine Erhöhung der Menge an Methansulfonsäure in einer weitergehenden verbesserten Aktivierung und Stabilisierung des eingesetzten bereits aktivierten Aluminiumsalzes.

Für die Flächenanteile der Peaks 1, 2 bis 3, 4 bis 8, 9 bis 11 und 12 und mehr an der Gesamtfläche aller für die jeweilige Probe im Chromatogramm erhaltenen Peaks für die Aktivierung eines bereits nicht aktivierten Aluminiumchlorohydrats Pulvers wurden folgende Werte erhalten:

| | Peak 1 | Peak 2 - 3 | Peak 4 - 8 | Peak 9 - 11 | Peak 12 und > 12 |
|---|---|---|---|---|---|
| V-II | 6 | 25 | 41 | 28 | 0 |
| E-III | 1 | 7 | 40 | 42 | 10 |

Die erfindungsgemäße Rezeptur E-IV weist einen signifikant höheren Anteil an kurzkettigen Polymeren (Peaks 9 bis 12 und mehr), d. h. eine deutlich bessere Aktivierung, auf als das Vergleichsbeispiel V-II. Weiterhin ist aus der obigen Tabelle ersichtlich, dass die Polymerverteilung durch die Erhöhung der Menge an Methansulfonsäure zugunsten der kurzkettigen Polymere verschoben werden kann. Folglich resultiert eine Erhöhung der Menge an Methansulfonsäure in einer weitergehenden verbesserten Aktivierung des eingesetzten nicht aktivierten Aluminiumsalzes.

### 2. In-vivo Test zur Antitranspirantwirkung

Zur Ermittlung der Antitranspirantwirkung wurde eine Antitranspirantstudie auf dem Rücken von 18 Probanden durchgeführt. Hierzu wurden die folgenden schweißhemmenden Mittel verwendet:

| Schweißhemmendes Mittel | Nr |
|---|---|
| Wässrige Lösung mit 10 % ACH | V-III |
| Wässrige Lösung mit 10 %* ACH und 1 %* Methansulfonsäure (S-Ia) | E-IV** |
| Wässrige Lösung mit 10 %* ACH und 2 %* Methansulfonsäure (S-Ia) | E-V** |
| W/O-Emulsionsbasis mit 15 %* ACH und 15 % Propandiol | V-IV |
| W/O-Emulsionsbasis mit 15 %* ACH, 15 % Propandiol, 2 %* Methansulfonsäure (S-Ia) | E-VI** |

| | |
|---|---|
| *Aktivsubstanz ** erfindungsgemäß | |

Auf dem Rücken von 18 Probandinnen im Alter von 35 bis 75 Jahren wurden jeweils auf einer Seite neben dem Rückgrat 40 µL der schweißhemmenden Mittel V-III, E-IV und E-V aufgetragen. Nach 5 Minuten wurden die behandelten Stellen mit okklusiver nichtadsorbierender Folie abgedeckt. Nach 2 Stunden wurden diese nichtadorbierenden Pads entfernt. Die Zusammensetzungen wurden an vier aufeinanderfolgenden Tagen jeweils auf die zuvor beschriebene Art und Weise auf den Rücken der Probandinnen aufgetragen. 24 h nach dem letzten Auftragen der Zusammensetzung wurden auf dem Rücken der Probandinnen saugfähige Pads an den Stellen aufgebracht, wo zuvor die Zusammensetzungen appliziert wurden. Weiterhin wurden auf der anderen Seite des Rückgrads in der gleichen Höhe ebenfalls Pads aufgebracht, welche als Kontrolle dienten. Nachdem die Probandinnen für ca. 15 Minuten bei 80 °C in der Sauna geschwitzt hatten, wurde die Menge des durch die Pads aufgenommenen Schweißes gravimetrisch bestimmt, wobei jede Zusammensetzung mit der jeweils korrespondierenden unbehandelten Stelle auf dem Rücken verglichen wurde. Aus der gravimetrischen Bestimmung der Schweißmenge wurde die Schweißreduktion ermittelt, wobei alle ermittelten Werte statistisch signifikant waren.

In einer zweiten Studie wurde auf dem Rücken von 18 Probandinnen im Alter von 35 bis 75 Jahren jeweils 40 µL der schweißhemmenden Mittel V-IV und E-VI aufgetragen und ebenso verfahren wie oben.

Die Schweißreduktion der jeweiligen Zusammensetzung im Vergleich zu einer unbehandelten Hautstelle ist in der nachfolgenden Tabelle wiedergegeben:

| Nr | Schweißreduktion |
|---|---|
| V-III | 47,5 % |
| E-IV** | 51,5 % |
| E-V** | 63,0 % |
| V-IV | 17,3 % |
| E-VI** | 30,4 % |

Durch Zusatz der Methansulfonsäure (S-Ia) wird die Schweißreduktion und damit auch die Antitranspirantwirkung signifikant gesteigert. Durch Einsatz einer höheren Menge der Methansulfonsäure ist eine weitergehende Schweißreduktion möglich. Somit resultiert durch den Zusatz der Methansulfonsäure eine signifikant verbesserte Aktivierung und Stabilisierung des schweißhemmenden Aluminiumsalzes und damit auch eine signifikant verbesserte Schweißreduktion der erfindungsgemäßen schweißhemmenden Mittel.

### 3. Formulierungen:

Die nachfolgenden Formulierungsbeispiele sollen den Gegenstand der Erfindung erläutern, ohne ihn hierauf zu beschränken. Die in den nachfolgenden Beispielen eingesetzte Verbindung der Formel (S-I) ist bevorzugt Methansulfonsäure bzw. deren Salze (S-Ia) sowie Mischungen der Methansulfonsäure und deren Salze oder Mischungen der Salze der Methansulfonsäure:
Schweißhemmende kosmetische Mittel (Mengenangaben in Gew.-%)

| | 1 | 2 | 3 | 4 | 5 | 6 |
|---|---|---|---|---|---|---|
| Hydrogenated Castor Oil | - | - | - | 1,5 | 1,5 | 1,5 |
| Stearyl Alcohol | 24,0 | 24,0 | 24,0 | 18 | 18 | 18 |
| Novata AB | - | - | - | 4 | 4 | 4 |
| Pulver aus ACH und Verbindung der Formel (S-I) (sprühgetrocknet, enthält 10 Gew.-% (S-I)) | 15,0 | 22,0 | 20,0 | 11,6 | 15,6 | 12,6 |
| Al-Zr-pentachlorohydrex Gly | 7,0 | - | - | 6,00 | - | - |
| PPG-14 Butyl Ether | 10,0 | 10,0 | 10,0 | 15,3 | 15,3 | 15,3 |
| gehärtetes Rizinusöl (z. B. Cutina HR) | 3,0 | 3,0 | 3,0 | - | - | - |
| Myristylmyristat | 1,5 | 1,5 | 1,5 | - | - | - |
| DL-Menthol | 0,2 | 0,2 | 0,2 | - | - | - |
| Eucalyptol | 0,2 | 0,2 | 0,2 | - | - | - |
| Anethol | 0,2 | 0,2 | 0,2 | - | - | - |
| Silica Dimethyl Silylate | 1,4 | 1,4 | 1,4 | - | - | - |
| Silica | 0,3 | 0,3 | 0,3 | - | - | - |
| Talkum | - | - | - | 3 | 3 | 3 |
| Emulgin B1 | - | - | - | 3 | 3 | 3 |
| Parfum | 2,0 | 2,0 | 2,0 | 1 | 1 | 1 |
| Cyclomethicone (mind. 95 Gew.-% Cyclopentasiloxan) | ad 100 | ad 100 | ad 100 | ad 100 | ad 100 | ad 100 |

Schweißhemmende kosmetische Mittel einer Öl-in-Wasser-Emulsion (Mengenangaben in Gew.-%)

| | 7 | 8 | 9 |
|---|---|---|---|
| Cutina ® AGS | 2,5 | 2,5 | 2,5 |
| Cutina® FS45 | 3,5 | 3,5 | 3,5 |
| Eumulgin® B2 | 0,8 | 0,8 | 0,8 |
| Eumulgin® B3 | 0,8 | 0,8 | 0,8 |
| Diisopropyladipat | 6,0 | 6,0 | 6,0 |
| Novata® AB | 4,0 | 4,0 | 4,0 |
| Cutina® CP | 5,0 | 5,0 | 5,0 |
| Cutina® HR | 4,0 | 4,0 | 4,0 |
| Kesterwachs K62 | 5,0 | 5,0 | 5,0 |
| Locron® L (ACH-Lösung 50%ig) | 40 | 40 | 30 |
| Talkum Pharma G | 10 | 10 | 10 |
| Parfüm | 1,2 | 1,2 | 1,2 |
| 2-Benzylheptan-1-ol | - | 0,3 | 0,3 |
| Sensiva SC 50 | 0,6 | 0,6 | 0,6 |
| Verbindung der Formel (S-I) | 2,0 | 3,0 | 3,0 |
| 1,2-Propandiol | 10 | 10 | 10 |
| Wasser, vollentsalzt | ad 100 | ad 100 | ad 100 |

Schweißhemmende kosmetische Mittel in Form einer Mikroemulsionen (Angaben in Gew.-%)

| | 10 | 11 | 12 | 13 |
|---|---|---|---|---|
| Plantaren® 1200 | 1,7 | 1,7 | - | - |
| Plantaren® 2000 | 1,1 | 1,4 | 2,4 | 2,4 |
| Glycerinmonooleat | 0,71 | 0,71 | - | - |
| Dioctylether | 4,0 | 4,0 | 0,090 | 0,090 |
| Octyldodecanol | 1,0 | 1,0 | 0,020 | 0,020 |
| Parfümöl | 1,0 | 1,0 | 1,0 | 1,0 |
| Aluminiumchlorohydrat | 8,0 | 5,0 | 5,0 | 10 |
| 1,2-Propylenglycol | 5,0 | 5,0 | - | - |
| Glycerin | - | - | 5,0 | 5,0 |
| 2-Benzylheptan-1-ol | 0,50 | - | - | - |
| Triethylcitrat | - | 0,50 | 0,50 | 0,50 |
| Triclosan | 0,10 | - | - | - |
| Verbindung der Formel (S-I) | 1,0 | 2,0 | 2,5 | 2,5 |
| Wasser | ad 100 | ad 100 | ad 100 | ad 100 |

Schweißhemmende kosmetische Mittel in Form von Roll-ons (Mengenangaben in Gew.-%)

| | 14 | 15 | 16 | 17 |
|---|---|---|---|---|
| Ethanol 96 %-ig,(DEP vergällt) | 30 | 30 | 28 | 28 |
| Mergital® CS 11 | 2,0 | 2,0 | - | - |
| Eumulgin® B3 | 2,0 | 2,0 | 2,0 | 2,0 |
| Emulgin® B1 | - | - | 2,0 | 2,0 |
| Aluminiumchlorohydrat 50 % (Locron L) | - | 20 | - | 16 |
| Al-Zr-pentachlorohydrex Gly | 20 | - | 16 | - |
| Hydroxyethylcellulose | 0,50 | 0,50 | 0,30 | 0,30 |
| Verbindung der Formel (S-I) | 2,5 | 0,50 | 2,0 | 1,5 |
| EDTA | - | - | - | 0,050 |
| Cocamidopropyl PG-Dimonium Chloride Phosphate | 0,20 | - | - | - |
| Parfümöl | 0,80 | 10,80 | 1,0 | 1,0 |
| Wasser | ad 100 | ad 100 | ad 100 | ad 100 |

### Antitranspirant-Tücher (Beispiele Nr. 18 bis 21)

Für die Ausführungsform als Antitranspirant-Tuch wurde ein einlagiges Substrat aus 100 % Viskose mit einem Flächengewicht von 50 g/m² mit jeweils 75 g der Beispielemulsionen 14 bzw. 15 pro Quadratmeter oder mit jeweils 75 g der Beispielzusammensetzungen 10 bzw. 11 beaufschlagt, in Tücher geeigneter Größe geschnitten und in Sachets verpackt.

Schweißhemmende kosmetische Mittel in Form einer Wasser-in-Öl-Emulsion (Mengenangaben in Gew.-%)

| | 22 | 23 | 24 |
|---|---|---|---|
| Aluminiumchlorohydrat 50% in Wasser (Locron L) | 35,6 | 35,6 | 20,0 |
| 1,2-Propylenglycol | 13,0 | 13,0 | 13,0 |
| Cyclohexasiloxan | 6,00 | 6,00 | 6,00 |
| Finsolv TN | 8,00 | 8,00 | 8,00 |
| Abil EM 90 | 1,20 | 1,20 | 1,20 |
| Polyethylen-Wachs (MW= 500 g/mol, Smp = 83 bis 91 °C) | 10,0 | 10,0 | 10,0 |
| Polyalphaolefin-Wachs (MW = 1800 g/mol, Smp = 41 °C) | 0,100 | 0,100 | 0,100 |
| Verbindung der Formel (S-I) | 2,00 | 0,500 | 0,500 |
| EDTA | - | 0,0500 | 0,0500 |
| Wasser | ad 100 | ad 100 | ad 100 |
| Parfum | 1,00 | 1,00 | 1,00 |

Schweißhemmende kosmetische Mittel (Mengenangaben in Gew.-%)

| | 25 | 26 |
|---|---|---|
| Cyclopentasiloxan | 14,0 | 14,0 |
| Abil EM 97 | 3,00 | 3,00 |
| Ethanol 96% | 10,0 | 10,0 |
| Aluminiumchlorohydrat 50% in Wasser (Locron L) | 40,0 | 40,0 |
| 1,2-Propylenglycol | 20,3 | 20,3 |
| Wasser | 11,6 | 11,6 |
| Verbindung der Formel (S-I) | 2,00 | 0,500 |
| EDTA | - | 0,0750 |
| Parfum | 1,00 | 1,00 |

Schweißhemmende kosmetische Mittel (Mengenangaben in Gew.-%, bezogen auf das Gesamtgewicht der treibmittelfreien Zusammensetzung)

| | 27 | 28 | 29 | 30 |
|---|---|---|---|---|
| Aluminiumchlorohydrate (ACH) | 28,6 | 14,29 | 32,11 | 28,57 |
| Bentone 38 V CG | 5,00 | 3,93 | 4,00 | 5,00 |
| Propylenecarbonate | 1,50 | 0,71 | 1,50 | 1,80 |
| Fragrance | 7,14 | 6,50 | 5,00 | 6,50 |
| 2-Ethylhexylpalmitate | - | 73,57 | - | - |
| Abil K 4 | 48,4 | - | - | - |
| Isopropylmyristate | 7,37 | - | 10,00 | 19,22 |
| Triethylcitrat | - | - | 10,5 | 19,2 |
| C10-C13 Isoalkane | - | - | 35,39 | 19,21 |
| Verbindung der Formel (S-I) | 2,00 | 1,00 | 1,50 | 0,500 |

Die Beispielzusammensetzungen 27 bis 30 wurden in eine gegebenenfalls mit Epoxy-Phenollack beschichtete Aluminiumspraydose in einem Gewichtsverhältnis von Treibmittel (Butan/Propan/Isobutangemisch) zu Suspension von 80:20 bzw. 85:15 bzw. 60:40 bzw. 90:10 abgefüllt.

Schweißhemmende kosmetische Mittel (Mengenangaben in Gew.-%, bezogen auf das Gesamtgewicht der treibmittelfreien Zusammensetzung)

| | 31 | 32 | 33 |
|---|---|---|---|
| Aluminiumchlorhydrat (ACH) | 33,0 | 33,0 | 33,0 |
| C₁₀-C₁₃-Isoalkan | 8,90 | 8,90 | 8,90 |
| Dow Corning ES-5227 DM | 1,40 | 1,40 | 1,40 |
| Isoceteth-20 | 0,500 | 0,500 | 0,500 |
| Dimethicone | 4,20 | 4,20 | 4,20 |
| Isopropylmyristat | 9,00 | 9,00 | 9,00 |
| 1,2 Propandiol | 7,00 | 25,0 | 25,0 |
| Phenoxyethanol | 0,500 | 0,500 | 0,500 |
| Parfüm | 2,50 | 2,50 | 2,50 |
| Verbindung der Formel (S-I) | 2,00 | 0,500 | 1,50 |
| L-Menthol | 0,400 | 0,300 | - |
| trans-Anethol | - | 0,300 | - |
| Eucalyptol | - | 0,300 | - |
| Wasser | ad 100 | ad 100 | ad 100 |

Die Beispielzusammensetzungen 31 bis 33 wurden in eine gegebenenfalls mit Epoxy-Phenollack beschichtete Aluminiumspraydose in einem Gewichtsverhältnis von Treibmittel (Butan/Propan/Isobutangemisch) zu W/O-Emulsion von 80:20 bzw. 85:15 bzw. 60:40 bzw. 90:10 abgefüllt.

Schweißhemmende kosmetische Mittel in Form von O/W-Emulsionen (Mengenangaben in Gew)

| | 34 | 35 | 36 |
|---|---|---|---|
| Aluminiumchlorhydrat (ACH) | 13,0 | 13,0 | 13,0 |
| Kalium Aluminiumsulfat KAl(SO₄)₂· 12 H₂O | 1,50 | 1,50 | 1,50 |
| Talk | 1,0 | - | - |
| Bentonite | - | 1,00 | - |
| Hectorite | - | - | 5,00 |
| Brij S 2 | 2,50 | 2,50 | 2,50 |
| Brij S 721 | 1,50 | 1,50 | 1,50 |
| Parfum | 1,10 | 1,10 | 1,10 |
| Arlamol E | 0,500 | 0,500 | 0,500 |
| Bisabolol | 0,100 | 0,100 | 0,100 |
| Dry Flo PC | 0,100 | 0,100 | 0,100 |
| Verbindung der Formel (S-I) | 2,00 | 3,00 | 1,00 |
| Dow Corning 2501 Cosmetic Wax | 0,100 | 0,100 | 0,100 |
| Tocopherylacetat | 0,100 | 0,100 | 0,100 |
| Wasser | ad 100 | ad 100 | ad 100 |

Schweißhemmende kosmetische Mittel (Mengenangaben in Gew.-%, bezogen auf das Gesamtgewicht der treibmittelfreien Zusammensetzung)

| | 37 | 38 | 39 | 40 |
|---|---|---|---|---|
| Aluminiumchlorhydrat (ACH) | 33,0 | 33,0 | 33,0 | 33,0 |
| Cyclomethicone | 12,0 | 9,40 | - | - |
| C10-C13-Isoalkan | - | - | 9,40 | 8,90 |
| Dow Corning ES-5227 DM | - | 1,40 | 1,40 | 1,40 |
| Abil EM 90 | 3,00 | - | - | - |
| Brij IC 20 | - | - | - | 0,500 |
| Dimethicone | - | 4,20 | 4,20 | 4,20 |
| Isopropylmyristat | 9,00 | 9,00 | 9,00 | 9,00 |
| Verbindung der Formel (S-I) | 2,50 | 1,00 | 3,00 | 0,500 |
| 1,2 Propandiol | 7,00 | 7,00 | 7,00 | 7,00 |
| Phenoxyethanol | 0,500 | 0,500 | 0,500 | 0,500 |
| Parfüm | 2,50 | 2,50 | 2,50 | 2,50 |
| Wasser | add 100 | add 100 | add 100 | add 100 |

Die Beispielzusammensetzungen 37 bis 40 wurden in eine gegebenenfalls mit Epoxy-Phenollack beschichtete Aluminiumspraydose in einem Gewichtsverhältnis von Treibmittel (Butan/Propan/Isobutangemisch) zu W/O-Emulsion von 80:20 bzw. 85:15 bzw. 60:40 bzw. 90:10 abgefüllt.

Schweißhemmende kosmetische Mittel in Form von W/O-Emulsionen (Mengenangaben in Gew.-%)

| | 41 | 42 | 43 | 44 |
|---|---|---|---|---|
| Aluminiumchlorohydrat 50% in Wasser (Locron L) | 62,5 | 62,5 | 60,0 | 58,0 |
| Propylenglycol | 5,00 | 5,00 | 7,50 | 9,50 |
| C12-C15 Alkylbenzoat | 8,04 | 8,04 | 8,04 | 8,04 |
| Dimethicone 2 cst | 6,43 | 6,43 | 6,43 | 6,43 |
| Dimethicone 5 cst | 1,57 | 1,57 | 1,57 | 1,57 |
| Polyethylen | 10,2 | 11,7 | 9,70 | 12,2 |
| Abil EM 90 | 0,998 | 0,998 | 0,998 | 0,998 |
| Abil EM 97 | 1,22 | 1,22 | 1,22 | 1,22 |
| Verbindung der Formel (S-I) | 2,50 | 1,00 | 3,00 | 0,500 |
| Synthetisches Wachs | 0,100 | 0,100 | 0,100 | 0,100 |
| Parfüm | 1,50 | 1,50 | 1,20 | 1,50 |

Es wurden die folgenden Handelsprodukte eingesetzt:

| Handelsprodukt | INCI | Lieferant/Hersteller |
|---|---|---|
| Abil EM 90 | CETYL PEG/PPG-10/1 Dimethicone | Evonik |
| Abil EM 97 | Bis-PEG/PPG-14/14 Dimethicone, Cyclomethicone | Evonik |
| Abil K4 | Cyclomethicone | Goldschmidt |
| Arlamol E | PPG-15 Stearyl ether | Croda |
| Bentone 38 V CG | Disteardimonium Hectorite | Elementis Specialities |
| Brij IC 20 | Isoceteth-20 | Croda |
| Brij S 2 | Steareth-2 | Croda |
| Brij S 721 | Steareth-21 | Croda |
| Cutina® CP | Cetyl Palmitate | BASF |
| Cutina® FS45 | Palmitic Acid, Stearic Acid | BASF |
| Cutina® HR | Hydrogenated Castor Oil | BASF |
| Dow Corning® 245 | Cyclopentasiloxan | Dow Corning |
| Dow Corning ® 2501 | Bis-PEG-18 Methyl ether dimethyl silane | Dow Corning |
| Dow Corning ES-5227 DM | Dimethicone, PEG/PPG-18/18 Dimethicone im Gewichtsverhältnis 3:1 | Dow Corning |
| Dry Flo PC | Aluminum Starch Octenylsuccinate | National Starch |
| Eumulgin® B1 | Ceteareth-12 | BASF |
| Eumulgin® B2 | Ceteareth-20 | BASF |
| Eumulgin® B3 | Ceteareth-30 | BASF |
| Kesterwachs K62 | Cetearyl Behenate | Koster Keunen |
| Finsolv TN | C12-15 Alkyl Benzoate | Innospec |
| Locron L (AS = 50 %) | Aluminum Chlorohydrate | Clariant |
| Mergital® CS 11 | Ceteareth-11 | BASF |
| Novata® AB | Cocoglycerides (Schmelzpunkt 30 - 32 °C) | BASF |
| Plantaren® 1200 | LAURYL GLUCOSIDE, ca. 50 % AS | BASF |
| Plantaren® 2000 | DECYL GLUCOSIDE, ca. 50 % AS | BASF |
| Sensiva® SC 50 | 2-Ethylhexylglycerinether | Schülke & Mayr |

## Patentansprüche

1. Verwendung mindestens einer Verbindung der Formel (S-I), 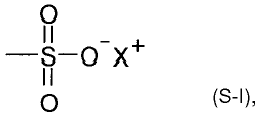 worin
X⁺ für H⁺, Li⁺, Na⁺, K⁺, NH₄⁺ oder ½ Mg²⁺ steht,
zur Aktivierung und/oder Stabilisierung mindestens eines schweißhemmenden Aluminiumsalzes.

2. Verwendung nach Anspruch 1, **dadurch gekennzeichnet, dass** die Verbindung der Formel (S-I) in einer Gesamtmenge von 0,01 bis 50 Gew.-%, vorzugsweise von 0,1 bis 40 Gew.-%, bevorzugt von 0,5 bis 30 Gew.-%, weiter bevorzugt von 1 bis 20 Gew.-%, noch weiter bevorzugt von 1,5 bis 10 Gew.-%, insbesondere von 2 bis 7 Gew.-%, bezogen auf das Gesamtgewicht aus der Verbindung der Formel (S-I), dem mindestens einen schweißhemmenden Aluminiumsalz sowie gegebenenfalls weiteren Inhaltsstoffen, verwendet wird.

## Claims

1. The use of at least one compound of formula (S-I) 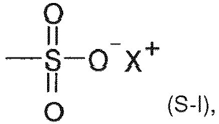 where
X⁺ stands for H⁺, Li⁺, Na⁺, K⁺, NH₄⁺ or ½ Mg²⁺,
for activating and/or stabilizing at least one antiperspirant aluminum salt.

2. The use according to claim 1, **characterized in that** the compound of formula (S-I) is used in a total amount of from 0.01 to 50 wt.%, preferably from 0.1 to 40 wt.%, more preferably from 0.5 to 30 wt.%, even more preferably from 1 to 20 wt.%, most preferably from 1.5 to 10 wt.%, in particular from 2 to 7 wt.%, based on the total weight of the compound of formula (S-I), the at least one antiperspirant aluminum salt and optionally additional ingredients.

## Revendications

1. Utilisation d'au moins un composé de la formule (S-I), 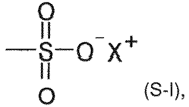 dans laquelle
X⁺ représente H⁺, Li⁺, Na⁺, K⁺, NH₄⁺ ou ½ Mg₂⁺,
destinée à activer et/ou à stabiliser au moins un sel d'aluminium anti transpirant.

2. Utilisation selon la revendication 1, **caractérisée en ce que** le composé de la formule (S-I) est utilisé dans une quantité totale de 0,01 à 50 % en poids, de préférence de 0,1 à 40 % en poids, de manière préférée de 0,5 à 30 % en poids, de manière davantage préférée de 1 à 20 % en poids, de manière encore davantage préférée de 1,5 à 10 % en poids, en particulier de 2 à 7 % en poids, rapporté au poids total issu du composé de la formule (S-I), de l'au moins un sel d'aluminium anti transpirant ainsi que le cas échéant d'autres ingrédients.
